# EUROPEAN PATENT SPECIFICATION

(11) **EP 3 479 865 B1**
(45) Date of publication and mention of the grant of the patent: **11.08.2021**
(21) Application number: 17820037.4
(22) Date of filing: 23.06.2017
(51) Int. Cl.: A61B 17/12

(54) **FILLING LIQUID, BALLOON DELIVERY DEVICE, MEDICAL ULTRASONIC DEVICE, MEDICAL SYSTEM, TUBULAR ORGAN BLOCKING TECHNIQUE, AND TUBULAR ORGAN BLOCKING RELEASE TECHNIQUE**
FÜLLFLÜSSIGKEIT, BALLONEINFÜHRUNGSVORRICHTUNG, MEDIZINISCHE ULTRASCHALLVORRICHTUNG, MEDIZINISCHES SYSTEM, TECHNIK ZUR BLOCKIERUNG TUBUSFÖRMIGER ORGANE UND TECHNIK ZUR LÖSUNG DER BLOCKIERUNG TUBUSFÖRMIGER ORGANE
LIQUIDE DE REMPLISSAGE, DISPOSITIF DE DISTRIBUTION DE BALLONNET, DISPOSITIF MÉDICAL À ULTRASONS, TECHNIQUE DE BLOCAGE D'ORGANE TUBULAIRE ET TECHNIQUE DE LIBÉRATION DE BLOCAGE D'ORGANE TUBULAIRE

(30) Priority: 27.06.2016 JP 2016126601
(43) Date of publication of application: 08.05.2019
(73) Proprietor: Chiba, Toshio, 156-0044 Tokyo (JP)
(72) Inventor: CHIBA Toshio, 1560044 Tokyo (JP); YAMASHITA Hiromasa, 1028275 Tokyo (JP); MOCHIZUKI Takashi, 2050014 Tokyo (JP); MARUYAMA Kazuo, 1738605 Tokyo (JP); SUZUKI Ryo, 1738605 Tokyo (JP)
(74) Representative: V.O.
(86) International application number: PCT/JP2017/023143
(87) International publication number: WO 2018/003680

(56) References cited:
- EP-A2- 1 991 052
- WO-A1-2010/068467
- FR-A1- 3 013 233
- JP-A- 2011 517 288
- JP-A- 2013 202 263
- JP-B1- 5 285 792
- US-A1- 2004 082 859
- US-A1- 2008 119 729
- US-A1- 2009 186 096
- US-A1- 2012 034 284
- US-A1- 2012 184 808

## Description

### Technical Field

The present disclosure relates to a system for terminating a tubular organ occlusion.

### Background Art

As a therapeutic method of congenital diaphragmatic hernia, tracheal occlusion, or occlusion of the fetal trachea with a balloon for a certain period of time has been proposed.

Congenital diaphragmatic hernia is an illness caused by a hole formed in the fetal diaphragm during the growth process of the fetus in the uterus, which allows intraperitoneal organs such as small intestine, large intestine, and stomach to migrate through the hole in the diaphragm to the thoracic cavity, resulting in an obstruction of growth of the fetal lungs. In an example of tracheal occlusion, a balloon is placed in the fetal trachea, the balloon is then inflated by liquid such as physiological saline solution injected into the balloon, and the balloon thus inflated occludes the fetal trachea. When the fetal trachea is kept occluded for a certain period of time, the pressure of the lung fluid stimulates the growth of the fetal lungs during the period and the intraperitoneal organs that entered the thoracic cavity are pushed back to the original positions in the peritoneal cavity. After that, the tracheal occlusion is terminated by deflating the balloon, in preparation for pulmonary respiration after birth.

A known method of terminating balloon tracheal occlusion is inserting an endoscope into the uterus and breaking the balloon with a guidewire or the like. As this technique involves endoscopic surgery, this technique is invasive to the human body and hence burdensome on the patient. Further, as endoscopic surgery needs to be performed by a medical specialist or the like, it is sometimes difficult to set up a system for that.

To address this, a technique using an ultrasonic wave has been proposed as a minimally invasive and easy method of terminating balloon tracheal occlusion. For example, an ultrasonic medical system recited in Patent Literature 1 includes a probe with an ultrasonic transducer and a transmitter that controls the probe in such a way as to form an ultrasonic transmission beam. The transmitter controls the probe in such a way as to form the transmission beam directed to the balloon placed in the tubular trachea. The probe transmits an ultrasonic wave along the transmission beam and causes the liquid filler that fills the balloon to evaporate due to the ultrasonic waves. This raises the pressure inside the balloon.

The balloon recited in Patent Literature 1 includes a backflow check valve that curtails the filler injected in the balloon from flowing out of the balloon. The backflow check valve stops curtailing the filler from flowing out when the pressure in the balloon rises. Because of this, when the pressure in the balloon rises due to the ultrasonic wave, the filler is discharged from the balloon through the backflow check valve. This deflates the balloon and hence terminates the balloon tracheal occlusion.

### Citation List

### Patent Literature

Patent Literature 1: Unexamined Japanese Patent Application Kokai Publication No. 2013-202263.

Patent Literature 2: US 2009/186096 A1, which discloses a method for the preparation of microencapsulated essential oils. The microcapsules containing essential oils or a formulation containing thereof may be used for various non-agricultural applications, i.e. in medical treatment.

Patent Literature 3: JP 5 285 792 B1, which discloses an ultrasonic probe, comprising a transducer for images and a transducer for vaporization. A transmission part controls the transducer for the vaporization of the ultrasonic probe, so as to form a transmission beam toward the position of a balloon and send ultrasonic waves for the vaporization. A liquid filler is filled inside the balloon, and the balloon closes the tubular tissue of a living body. By sending the ultrasonic waves to the balloon, the liquid filler filled inside the balloon is vaporized by the ultrasonic waves and discharged from the inside of the balloon, the balloon is shrunk, and the closure of the tubular tissue is canceled.

### Summary of Invention

### Technical Problem

However, in the conventional ultrasonic medical system, the backflow check valve may fail to stop curtailing the filler from flowing out, resulting in a failure to terminate the balloon tracheal occlusion.

The present disclosure is made in view of the above-described circumstances and has an objective of providing a method for occluding tubular organ and a method for terminating tubular organ occlusion that can terminate a balloon occlusion of a tubular organ such as trachea in a minimally invasive, easy, and assured way as well as a filling liquid, a balloon delivery device, a medical ultrasonic device, and a medical system for these methods, which are not part of the invention.

### Solution to Problem

To achieve the above-described objective, a system is provided, comprising a balloon, comprising latex, and a filling liquid for filling a balloon for a method for occluding a tubular organ, the filling liquid comprising a microcapsule in which a naturally derived essential oil, that dissolves the balloon, is encapsulated, wherein the microcapsule is destroyable by an ultrasonic beam; and a liquid that is harmless in the tubular organ and does not dissolve the balloon.

The system may also comprise a balloon delivery device, according to a second aspect of the present disclosure including:
a catheter to which a balloon is attachable at a front end of the catheter; and
injection means for injecting the filling liquid to a tubular tissue through the catheter.

The system may also comprise a medical ultrasonic device according to a third aspect of the present disclosure including:
a probe including an ultrasonic transducer; and
an ultrasonic wave control unit that controls the ultrasonic transducer to cause the probe to emit an ultrasonic beam that destroys the microcapsule to the balloon filled with the filling liquid according to the first aspect of the present disclosure and occluding a tubular organ.

A medical system, which is not part of the invention, includes:
the balloon delivery device according to the second aspect of the present disclosure; and
the medical ultrasonic device according to the third aspect of the present disclosure.

A method for occluding a tubular organ, which is not part of the invention, includes:
a step of preparing a catheter with a balloon attached to a front end of the catheter;
a step of delivering the balloon into a certain tubular organ by means of the catheter;
a step of filling the balloon with the filling liquid according to the first aspect of the present disclosure by means of the catheter, inflating the balloon, and occluding the tubular organ with the inflated balloon; and
a step of detaching the catheter from the balloon.

A method for terminating tubular organ, which is not part of the invention, includes
a step of applying the ultrasonic beam to a balloon that is filled with the filling liquid according to the first aspect of the present disclosure and occludes a tubular organ and of breaking the balloon as a result.

### Advantageous Effects of Invention

According to the present disclosure, a balloon is filled with a filling liquid containing microcapsules, a balloon-dissolving substance that dissolves the balloon is encapsulated in the microcapsules, and an ultrasonic wave control unit causes a probe to emit an ultrasonic beam that destroys the microcapsules in the filling liquid in the balloon when applied to the balloon occluding the tubular organ. This makes it possible to destroy the microcapsules in the filling liquid in the balloon by simply applying the ultrasonic beam from the probe to the balloon occluding the tubular organ and to dissolve and break the balloon with the balloon-dissolving substance let out of the destroyed microcapsules. Therefore, it is possible to provide a medical system and a medical ultrasonic device that can terminate a balloon occlusion of a tubular organ in a minimally invasive, easy, and assured way.

### Brief Description of Drawings

FIG. 1 is a view illustrating a configuration of a medical system according to an embodiment of the present disclosure;
FIG. 2 is a diagram illustrating a functional configuration of an endoscope control unit according to an embodiment;
FIG. 3 is a diagram illustrating a functional configuration of an ultrasonic wave control unit according to an embodiment;
FIG. 4 is an enlarged view of the vicinity of the thoracic cavity of a fetus affected by congenital diaphragmatic hernia;
FIG. 5 is a view illustrating a balloon placed in the fetal trachea by using endoscopic device;
FIG. 6 is a view illustrating a trachea occluded by the balloon inflated by filling liquid that fills the balloon;
FIG. 7 is a view illustrating the balloon detached from the endoscopic device, the balloon occluding the trachea;
FIG. 8 is an enlarged view of the vicinity of the fetal thoracic cavity, intraperitoneal organs pushed back out of the thoracic cavity due to the tracheal occlusion;
FIG. 9 is a view illustrating a first ultrasonic beam applied to the balloon;
FIG. 10A is an enlarged view illustrating the balloon and the fetal trachea during the termination of the balloon tracheal occlusion at the time of starting the application of the first ultrasonic beam;
FIG. 10B is an enlarged view illustrating the balloon and the fetal trachea during the termination of the balloon tracheal occlusion, microcapsules broken and limonene let out to the filling liquid;
FIG. 10C is an enlarged view illustrating the balloon and the fetal trachea during the termination of the balloon tracheal occlusion, the balloon broken by the effect of limonene and filling liquid let out of the balloon; and
FIG. 10D is an enlarged view illustrating the balloon and the fetal trachea during the termination of the balloon tracheal occlusion, the balloon deflated and the tracheal occlusion terminated.

### Description of Embodiments

An embodiment of the present disclosure will be described below with reference to the drawings. Like elements will be denoted by like reference signs throughout the drawings.

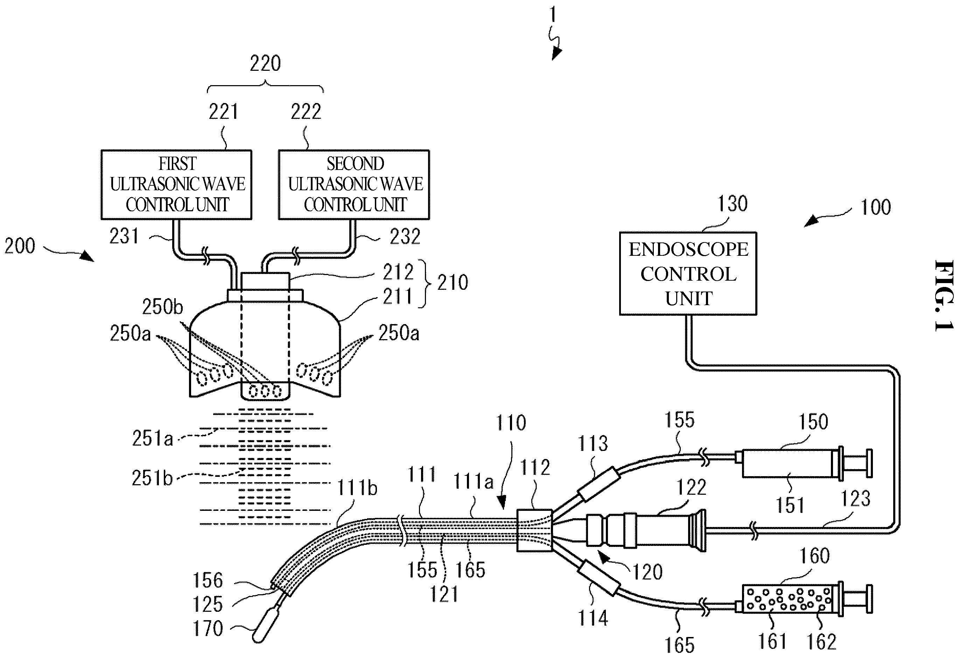

A medical system 1 according to an embodiment of the present disclosure is a system for occluding a tubular organ of a living body with a balloon 170 and for terminating the occlusion, the configuration of which is illustrated in FIG. 1. The medical system 1 includes an endoscopic device 100 and an ultrasonic device 200. Note that the proportions of the parts in FIG. 1 are altered as appropriate for easier understanding.

### (Configuration of the endoscopic device 100)

The endoscopic device 100 is a device for occluding a tubular organ of a living body with a balloon 170. The endoscopic device 100 includes a sheath 110, an endoscope 120, an endoscope control unit 130, a first syringe 150, a first catheter 155, a second syringe 160, and a second catheter 165.

The sheath 110 is a member used for allowing easy insertion of the insertion part 121 of the endoscope 120, the first catheter 155, and the second catheter 165 to the body. The sheath 110 includes a sheath body 111 and a connecter 112.

The sheath body 111 is a long tubular part in which a through inner hole is defined in the longitudinal direction, into which the insertion part 121 of the endoscope 120, the first catheter 155, and the second catheter 165 can be inserted. The sheath body 111 is made of rigid material such as metal or hard resin. The rear part 111a of the sheath body 111 is approximately linearly formed. In contrast, the front part 111b of the sheath body 111 is curved gently, approximately in a circular arc, in order to ease insertion into a living body. Note that the sheath body 111 may have individual through inner holes respectively having inside diameters large enough to insert the insertion part 121 of the endoscope 120, the first catheter 155, and the second catheter 165.

The connecter 112 is a part coupled to the rear end of the sheath body 111 to detachably connect the endoscope 120 with the sheath 110. The connecter 112 has an insertion hole defined therein that communicates with the through inner hole of the sheath body 111. The connecter 112 connects the operation part 122 of the endoscope 120 with the sheath body 111 by fitting or screwing, such that the insertion part 121 of the endoscope 120 is inserted into the sheath body 111 through the insertion hole. The connecter 112 further includes an insertion port part 113 having an insertion hole through which the first catheter 155 can be inserted and an insertion port part 114 having an insertion hole through which the second catheter 165 can be inserted. The first catheter 155 and the second catheter 165 are inserted to the through inner hole of the sheath body 111 respectively through the insertion port parts 113 and 114.

The endoscope 120 is a device for making observation inside a living body. The endoscope 120 includes an insertion part 121, an operation part 122, and a wire 123.

The insertion part 121 is an approximately cylindrical part to be inserted into a living body. The insertion part 121 is inserted into a living body, for example, with the sheath 110 attached to the endoscope 120. The insertion part 121 is flexible and, when the sheath 110 is attached to the endoscope 120, the insertion part 121 can be inserted into the sheath body 111 along the shape of the sheath body 111. Further, the insertion part 121 has a length approximately equal to that of the sheath body 111 and, when the sheath 110 is attached to the endoscope 120, the front end of the insertion part 121 does not protrude from the front end of the sheath body 111 but is slightly retreated in the sheath body 111. Further, an imaging unit 125 for taking images inside the living body is provided at the front part of the insertion part 121.

The imaging unit 125 includes, for example, a camera that includes an objective lens that condenses light from the subject, a charge-coupled device (CCD) image sensor, a complementary metal oxide semiconductor (CMOS) image sensor, and the like, and a lighting device that includes a light emitting diode (LED) and the like.

The operation part 122 is a part connected to the rear end of the insertion part 121 and is held by a user to operate the endoscope 120. The operation part 122 includes a switch, a knob, and the like (not illustrated) for operating various functions of the endoscope 120. Herein, the user is typically a doctor.

Note that the insertion part 121 may include in the insertion part 121 a cable wire and the like for changing the direction of the imaging unit 125 in such a way as to enable the user to direct the imaging unit 125 provided at the front part of the insertion part 121 in a desired direction to change the scope of images to be taken, by adjusting the length of the cable wire by operating a nob or the like provided on the operation part 122.

The wire 123 extends through the insertion part 121 and electrically connects the imaging unit 125 with the endoscope control unit 130. The imaging unit 125 and the endoscope control unit 130 transmit and receive various signals to and from each other through the wire 123.

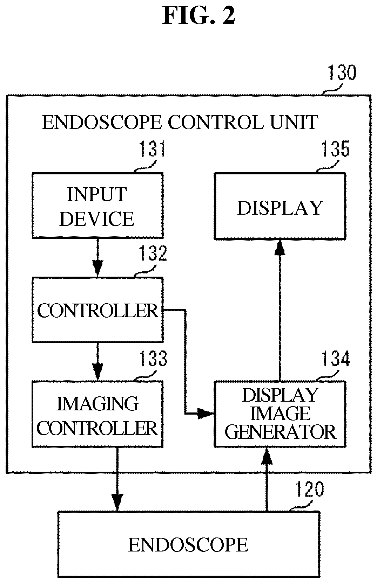

The endoscope control unit 130 is a unit for controlling the endoscope 120. The endoscope control unit 130, the functional configuration of which is illustrated in FIG. 2, includes an input device 131, a controller 132, an imaging controller 133, a display image generator 134, and a display 135.

The input device 131 is operated by the user to input an instruction to start or end operation of the imaging unit 125 and the like as well as to input various setting values and the like. The input device 131 includes, for example, a button, a switch, and the like, physically. Note that the input device 131 together with the display 135 may form a touch panel.

The controller 132 controls the imaging controller 133, the display image generator 134, and the like in accordance with operations on the input device 131, signals from various sensors (not illustrated), and the like.

The imaging controller 133 controls the imaging unit 125 under the control of the controller 132. For example, when the imaging controller 133 acquires an instruction from the controller 132 in response to an operation on the input device 131, the imaging controller 133 starts or ends an operation of the imaging unit 125 in accordance with the acquired instruction. Further, for example, the imaging controller 133 controls the operation of the imaging unit 125 in accordance with setting values and the like, the setting value designated by means of the controller 132 on the basis of an operation on the input device 131. The operation of the imaging unit 125 includes, for example, taking an image with the camera, emitting light from the lighting device, and the like.

Under the control of the controller 132, the display image generator 134 generates display data by acquiring image data indicating an image taken by the camera of the imaging unit 125 from the imaging unit 125 and converting the image data into display data for displaying the image on the display 135. Such conversion processing includes, for example, conversion processing of the image data acquired from the camera of the imaging unit 125 into pixel data indicating the pixel values for the display 135, noise removal processing, and the like.

The controller 132, the imaging controller 133, and the display image generator 134 physically include, for example, one or more processors, random access memory (RAM), read only memory (ROM), flash memory, dedicated electric circuits, and the like, which may be combined as appropriate.

The display 135 acquires display data from the display image generator 134 and displays the image represented by the display data. The display 135 may physically include, for example, a liquid crystal panel, a liquid crystal drive circuit, a back panel, and the like.

The first syringe 150 is an instrument used as injection means for injecting flush liquid 151. The first syringe 150 is attached to the rear end of the first catheter 155 with the cylinder of the first syringe 150 filled with the flush liquid 151. The flush liquid 151 may be, for example, physiological saline solution. When the endoscope 120 is inserted into the body, the vision of the endoscope 120 may be affected by turbidity of body liquid (amniotic fluid and the like) and the like. In such a case, the user manually operates the plunger of the first syringe 150 to inject the transparent flush liquid 151, causes the flush liquid 151 to spout from the front end of the first catheter 155, and thereby obtains appropriate vision of the endoscope 120.

The first catheter 155 is a tubular member to be inserted into the living body in order to inject flush liquid 151 into the living body. The first catheter 155 is installed by insertion from the insertion port part 113 provided for the connecter 112 through the through inner hole defined through the sheath body 111 in the longitudinal direction, and is inserted to the living body with the sheath 110. The flush liquid 151 filling the first syringe 150 attached to the rear end of the first catheter 155 goes through the first catheter 155 and spouts from the spout opening 156 provided at the front end of the first catheter 155.

The second syringe 160 is an instrument used as injection means for injecting filling liquid 161. The second syringe 160 is attached to the rear end of the second catheter 165 with the cylinder of the second syringe 160 filled with the filling liquid 161. The user manually operates the plunger of the second syringe 160 to inject the filling liquid 161 to the balloon 170 attached to the front end of the second catheter 165 and thereby inflates the balloon 170.

The filling liquid 161 is a liquid with which to fill the balloon 170 in order to inflate the balloon 170 in a tubular organ to occlude the tubular organ. The filling liquid 161 includes microcapsules 162 in which a material that dissolves the balloon 170 is encapsulated. The filling liquid 161 may be, for example, physiological saline solution mixed with a large number of limonene-encapsulating microcapsules 162. Herein, a microcapsule means a fine particle in which a substance, for example, a liquid is encapsulated within a thin membrane and includes, for example, a macromolecular micelle, a liposome, an emulsion, and the like. Microcapsules 162 having a particle diameter of about 1 to 100 µm are suitable for the use but the particle diameter of the microcapsules 162 is not limited to this range. Note that it is desirable that the filling liquid 161 containing the microcapsules 162 have such a viscosity and a density as to allow the filling liquid 161 to be easily injected to the balloon 170 through the second catheter 165.

For example, when liposomes are used as microcapsules 162, limonene-encapsulating liposomes can be easily produced by the Bangham method or the like. More specifically, phospholipid, which is a component of liposome layers, is dissolved in organic solvent (for example, mixed solvent of methanol and chloroform) and the organic solvent is removed by evaporation using an evaporator, thereby forming a thin membrane. Next, the limonene to be encapsulated and physiological saline solution are added to swell the thin membrane and, further, ultrasonic processing is applied to make the particles smaller and uniform, and thus limonene-encapsulating liposomes (microcapsules 162) are produced.

The second catheter 165 is a tubular member used for injecting the filling liquid 161 into the balloon 170. The second catheter 165 with the balloon 170 attached to the front end thereof is provided from the insertion port part 114 provided for the connecter 112 and through the through inner hole defined through the sheath body 111 in the longitudinal direction, and is inserted to the living body with the sheath 110. The filling liquid 161 filling the second syringe 160 attached to the rear end of the second catheter 165 is injected through the second catheter 165 to the balloon 170 attached to the front end of the second catheter 165.

The balloon 170 is a member in the form of a bag for occluding a tubular organ and made mainly of, for example, latex. The balloon 170 is detachably attached to the front end of the second catheter 165. More specifically, the balloon 170 includes a backflow check valve (not illustrated), which allows the filling liquid 161 to go inside and prevents the filling liquid 161 injected inside from going outside. The balloon 170 is attached to the second catheter 165 by sticking the needle (not illustrated) provided at the front end of the second catheter 165 into the backflow check valve.

### (Configuration of the ultrasonic device 200)

The ultrasonic device 200 is a device for terminating the occlusion of a tubular organ by the balloon 170 by using ultrasonic waves. The ultrasonic device 200 includes a probe 210, which includes a first probe 211 and a second probe 212, a control unit 220, which includes a first ultrasonic wave control unit 221 and a second ultrasonic wave control unit 222, a wire 231 electrically connecting the first probe 211 with the first ultrasonic wave control unit 221, and a wire 232 electrically connecting the second probe 212 with the second ultrasonic wave control unit 222.

The first probe 211 is an appliance that transmits, through the front part thereof which contacts the living body, a first ultrasonic beam 251a that breaks the microcapsules 162 in the balloon 170. The first probe 211 includes a plurality of first ultrasonic transducers 250a as illustrated in FIG. 1.

The first probe 211 is an annular member with a round through hole defined approximately in the center thereof. The front part of the first probe 211 has a sloped surface formed conically and concavely toward the round through hole. The plurality of first ultrasonic transducers 250a are arranged in order inside the sloped surface. Such arrangement of the plurality of first ultrasonic transducers 250a allows the first probe 211 to transmit the first ultrasonic beam 251a to a target located on the central axis of the round through hole (the central axis of the probe).

The second probe 212 is an appliance that transmits and receives, through the front part thereof which contacts the living body, a second ultrasonic beam 251b to obtain a sonogram, which is an image of the inside of the living body. The second probe 212 includes a plurality of second ultrasonic transducers 250b as illustrated in FIG. 1.

The second probe 212 is a columnar member inserted to the round through hole defined in the first probe 211 and detachably attached to the first probe 211. The first probe 211 and the second probe 212 are used usually with the second probe 212 attached to the first probe 211. The plurality of second ultrasonic transducers 250b are arranged in order inside the front part of the second probe 212.

Note that the forms of the first probe 211 and the second probe 212 and the arrangements of the ultrasonic transducers 250a and 250b are not limited to this and may be altered as appropriate. Further, the first probe 211 and the second probe 212 may include a damper, an acoustic matching layer, an acoustic lens, and the like (not illustrated). A damper is a member that curtails the ultrasonic waves produced by the ultrasonic transducers 250a and 250b from being transmitted backward. An acoustic matching layer is a member for decreasing the difference in acoustic impedance between the ultrasonic transducers 250a and 250b and the living body. An acoustic lens is a device that focuses the ultrasonic beam 251a and 251b, which will be described later.

Each of the ultrasonic transducers 250a and 250b is a device having a piezoelectric effect, including, for example, zirconate titanate, poly(vinylidene fluoride), quartz crystal, or the like and produces an ultrasonic wave by vibrating in response to an electric signal applied through the wire 231 or 232.

In particular, the plurality of first ultrasonic transducers 250a are devices to emit a first ultrasonic beam 251a that breaks microcapsules 162 in the filling liquid 161 when applied to the balloon 170 filled with the filling liquid 161 and occluding a tubular organ. The plurality of first ultrasonic transducers 250a generate an ultrasonic waves by vibrating in response to an electric signal applied thereto through the wire 231. The ultrasonic waves are emitted from the front part of the first probe 211 as a first ultrasonic beam 251a.

Note that it is preferable that the first ultrasonic beam 251a be an ultrasonic beam having a higher energy than that of the second ultrasonic beam 251b to ensure destruction of the microcapsules 162 and, for example, a line-focused ultrasonic beam is preferably used.

The plurality of second ultrasonic transducers 250b are devices for transmitting and receiving a second ultrasonic beam 251b to obtain a sonogram taken inside the living body.

The plurality of second ultrasonic transducers 250b produce ultrasonic waves by vibrating in response to an electric signal. The ultrasonic wave is emitted from the front part of the second probe 212 as a second ultrasonic beam 251b. Further, upon receiving the second ultrasonic beam 251b reflected inside the living body, the plurality of second ultrasonic transducers 250b output an electrical signal in accordance with the received second ultrasonic beam 251b as a reception signal.

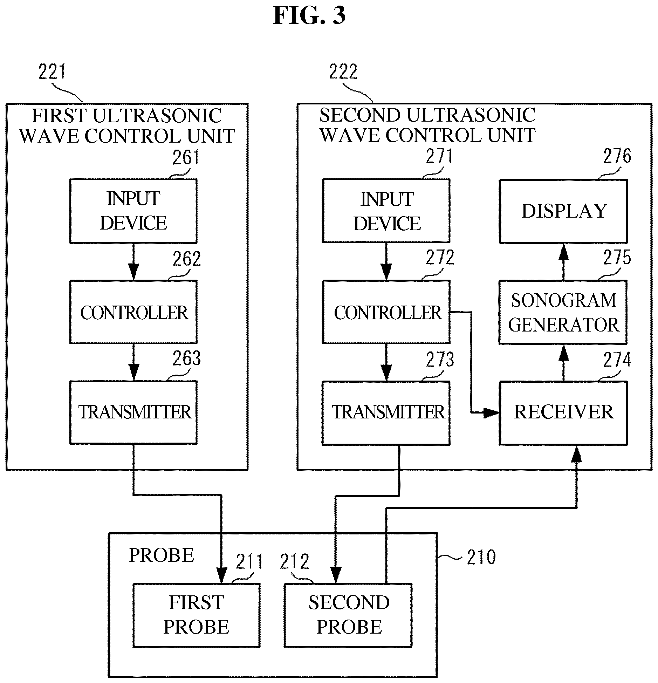

The first ultrasonic wave control unit 221 is a unit that controls the first probe 211 and includes an input device 261, a controller 262, and a transmitter 263, as FIG. 3 illustrates the functional configuration thereof.

The input device 261 is operated by the user for inputting an instruction to start or finish the emission of the first ultrasonic beam 251a, setting values of various kinds, and the like. The input device 261 physically includes, for example, a button, a switch, and the like.

The controller 262 controls the transmitter 263 in accordance with the operation on the input device 261, signals from various sensors (not illustrated), and the like.

Under the control by the controller 262, the transmitter 263 controls the plurality of first ultrasonic transducers 250a to cause the first probe 211 to emit the first ultrasonic beam 251a.

In particular, the transmitter 263 outputs a transmission signal, which is an electric signal, through the wire 231 to each of the plurality of first ultrasonic transducers 250a to control the plurality of first ultrasonic transducers 250a. Each of the plurality of first ultrasonic transducers 250a thereby acquires the transmission signal from the transmitter 263 and produces an ultrasonic wave in accordance with the acquired signal. The individual ultrasonic waves produced by the respective first ultrasonic transducers 250a compose a first ultrasonic beam 251a, which is emitted from the first probe 211.

The second ultrasonic wave control unit 222 is a unit that controls the second probe 212 and includes an input device 271, a controller 272, a transmitter 273, a receiver 274, a sonogram generator 275, and a display 276.

The input device 271 is operated by the user for inputting an instruction to start or finish the emission of the second ultrasonic beam 251b; an instruction to start or finish the display of a sonogram; setting values of various kinds; and the like. The input device 271 physically includes, for example, a button, a switch, and the like. Note that the input device 271 together with the display 276 may form a touch panel.

The controller 272 controls the transmitter 273, the receiver 274, and the like in accordance with the operation on the input device 271, signals from various sensors (not illustrated), and the like.

Under the control by the controller 272, the transmitter 273 controls the plurality of second ultrasonic transducers 250b to cause the second probe 212 to emit the second ultrasonic beam 251b.

In particular, the transmitter 273 outputs a transmission signal, which is an electric signal, through the wire 232 to each of the plurality of second ultrasonic transducers 250b to control the plurality of second ultrasonic transducers 250b. Each of the plurality of second ultrasonic transducers 250b thereby acquires the transmission signal from the transmitter 273 and produces an ultrasonic wave in accordance with the acquired signal. The individual ultrasonic waves produced by the respective first ultrasonic transducers 250b compose a first ultrasonic beam 251b, which is emitted from the first probe 212.

Under the control by the controller 272, the receiver 274 generates echo data in accordance with the second ultrasonic beam 251b reflected inside the living body on the basis of the reception signals outputted by the plurality of second ultrasonic transducers 250b. In particular, the receiver 274 generates the echo data by acquiring the reception signals outputted by the plurality of second ultrasonic transducers 250b through the wire 232 and applying phasing addition processing and the like to the acquired reception signals.

The sonogram generator 275 generates display data to display a sonogram on the display 276 on the basis of the echo data generated by the receiver 274. In particular, the sonogram generator 275 generates the display data by acquiring the echo data generated by the receiver 274 from the receiver 274 and applying appropriate image processing to the acquired echo data. The image processing herein is, for example, processing for allowing the display 276 to display an image of a predetermined mode, such as B-mode image or 2D image.

The controller 272, the transmitter 273, the receiver 274, and the sonogram generator 275 may physically include, for example, one or more processors, RAM, ROM, flash memory, dedicated electric circuits, and the like, which may be combined as appropriate.

The display 276 displays the sonogram represented by the display data generated by the sonogram generator 275. In particular, the display 276 acquires the display data from the sonogram generator 275 and displays the sonogram represented by the acquired display data. The display 276 may physically include, for example, a liquid crystal panel, a liquid crystal drive circuit, a back panel, and the like.

The configuration of the medical system 1 according to an embodiment has been described above. An operation of the medical system 1 according to the present embodiment will be described below.

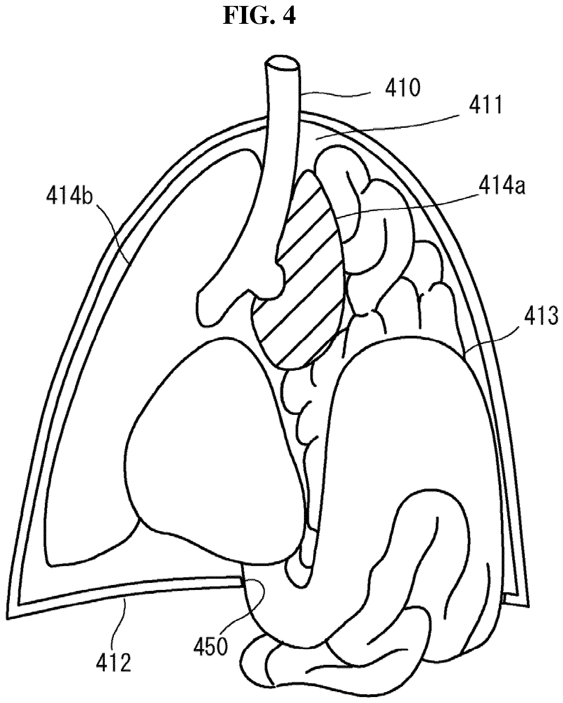

As to the present embodiment, an operation of the medical system 1 will be described with an example of application thereof to a treatment of congenital diaphragmatic hernia. Congenital diaphragmatic hernia is an illness in which the diaphragm of a fetus 412 has a hole 450, through which intraperitoneal organs 413 such as small intestine, large intestine, and stomach migrate into the thoracic cavity 411, obstructing the growth of the fetal lungs 414a and 414b, as illustrated in FIG. 4, which is an enlarged view of the vicinity of the fetal thoracic cavity 411. FIG. 4 illustrates an example in which the growth of the left lung 414a, shaded by oblique lines, is obstructed.

Note that the medical system 1 can be suitably used not only for the treatment of congenital diaphragmatic hernia but also for the treatment of various illnesses in which it is effective to occlude a tubular organ of the living body with a balloon 170 and to terminate the occlusion. Further, the living body is not limited to a living human body but may be a living animal body.

### (Occlusion of a tubular organ)

The user starts the operation of the imaging unit 125 by operating the input device 131 of the endoscope control unit 130. This causes the image taken by the camera of the imaging unit 125 to be displayed on the display 135.

In particular, the imaging controller 133 receives an instruction from the controller 132 to start operation of the imaging unit 125, based on the operation of the input device 131. In response to the instruction, the imaging controller 133 causes the light of the imaging unit 125 to emit light and causes the camera of the imaging unit 125 to take an image. The camera of the imaging unit 125 outputs image data that represent the image taken. The display image generator 134 acquires the image data from the imaging unit 125 and generates display data based on the acquired image data. The display 135 acquires the display data from the display image generator 134 and displays the image represented by the acquired display data.

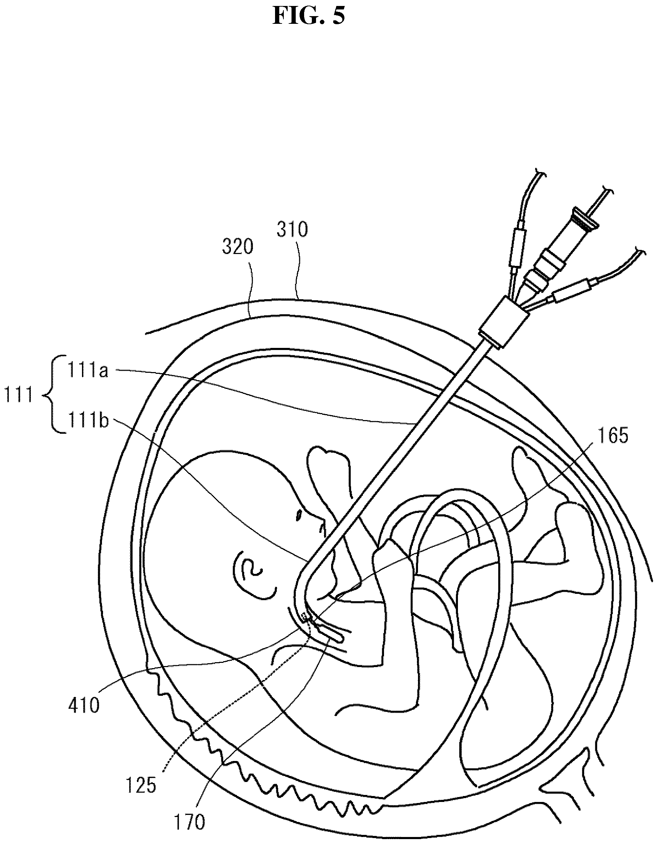

Holding the operation part 122, the user inserts the sheath body 111 into the uterus 320 through an insertion hole formed in the maternal abdomen 310, as illustrated in FIG. 5, wherein the insertion part 121, the first catheter 155, and the second catheter 165 are arranged in the sheath body 111 by being inserted therethrough in advance (see FIG. 1). The user at this time inserts the sheath body 111 into the uterus 320 while the balloon 170 attached to the front end of the second catheter 165 is not exposed at the front end of the sheath body 111 but housed in the sheath body 111.

The user inserts the front part 111b of the sheath body 111 into the fetal trachea 410, which is the target tubular organ to be occluded, by operating the operation part 122 while viewing the image displayed on the display 135. When the user has a poor vision in the endoscope 120 during the insertion of the sheath body 111, the user causes the flush liquid 151 to spout from the front end of the sheath body 111 as appropriate by operating the first syringe 150. When the front part 111b of the sheath body 111 reaches the fetal trachea 410, the user pushes the second catheter 165 in the direction of insertion and disposes the balloon 170 in such a way that the balloon 170 is exposed at the front end of the sheath body 111 (see FIG. 5).

While checking the state of inflation of the balloon 170 on the image taken by the endoscope 120 and displayed on the display 135, the user injects the filling liquid 161 into the balloon 170 by manually operating the second syringe 160 (by pushing down the plunger).

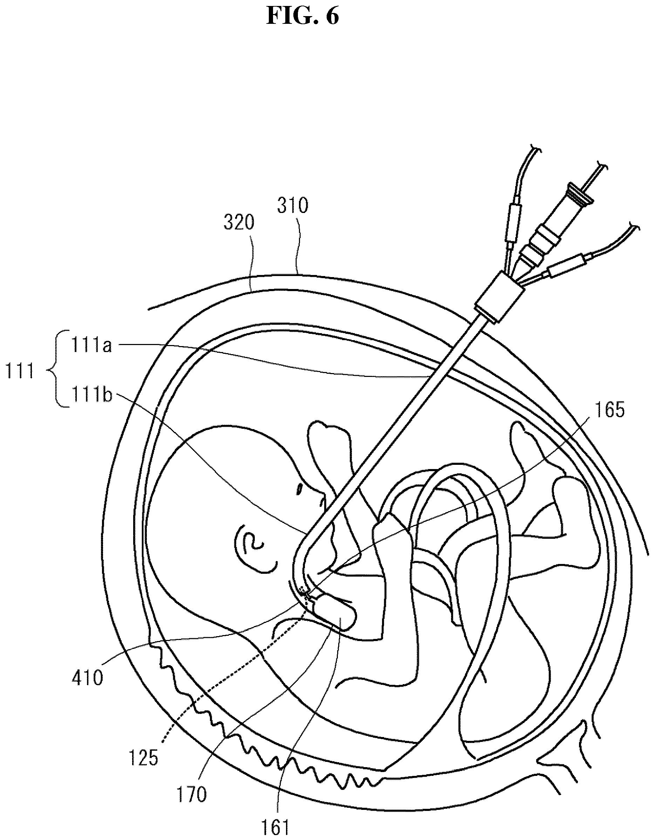

When the balloon 170 filled with the filling liquid 161 inflates and occludes the trachea 410 (see FIG. 6), the user stops operating the second syringe 160 and stops injecting the filling liquid 161 to the balloon 170.

The user pulls on the second catheter 165 while holding the operation part 122 to pull out the second catheter 165 alone from the sheath 110 with the sheath body 111 remaining in the maternal body. At this time, since the balloon 170 occludes the trachea 410, the balloon 170 is pressed against the trachea 410 while applying a certain force on the trachea 410. Thus, the balloon 170 is detached from the front end of the second catheter 165 simply by pulling out the second catheter 165.

Note that, due to the function of the backflow check valve of the balloon 170, the filling liquid 161 does not flow out of the balloon 170 but remains in the balloon 170 even when the second catheter 165 is pulled out.

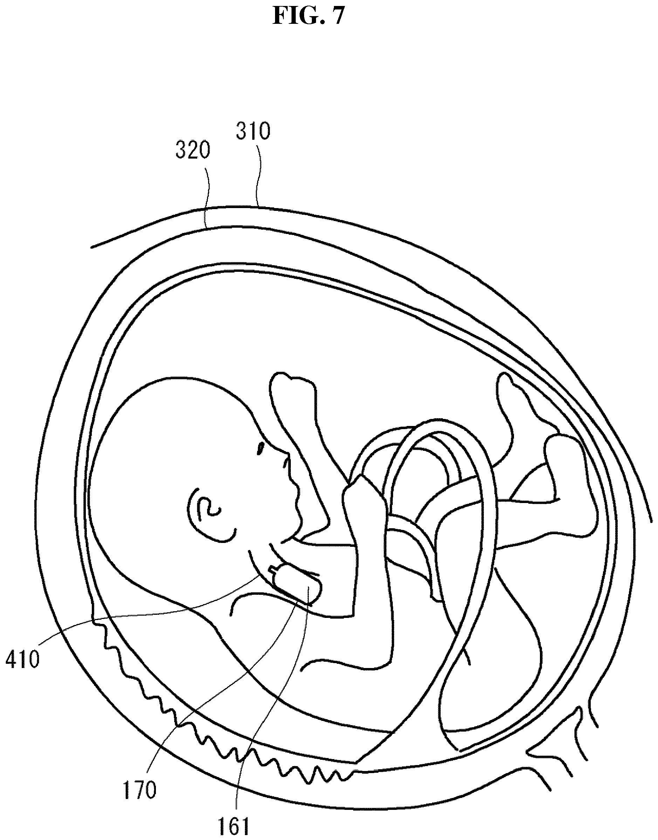

After checking the state of inflation of the balloon 170 on the image taken by the endoscope 120 and displayed on the display 135 and checking that the occlusion of the fetal trachea 410 is maintained, the user pulls the operation part 122 to pull out from the maternal body the sheath body 111, wherein the insertion part 121 of the endoscope 120 and the first catheter 155 remain inserted through the sheath body 111 (see FIG. 7).

The user stops the operation of the imaging unit 125 by operating the input device 131 of the endoscope control unit 130. In particular, the imaging controller 133 receives from the controller 132 an instruction to stop the operation of the imaging unit 125, based on the operation of the input device 131. In response to the instruction, the imaging controller 133 stops the light of the imaging unit 125 emitting light and stops the camera taking an image. This concludes the surgical treatment for occluding the fetal trachea 410.

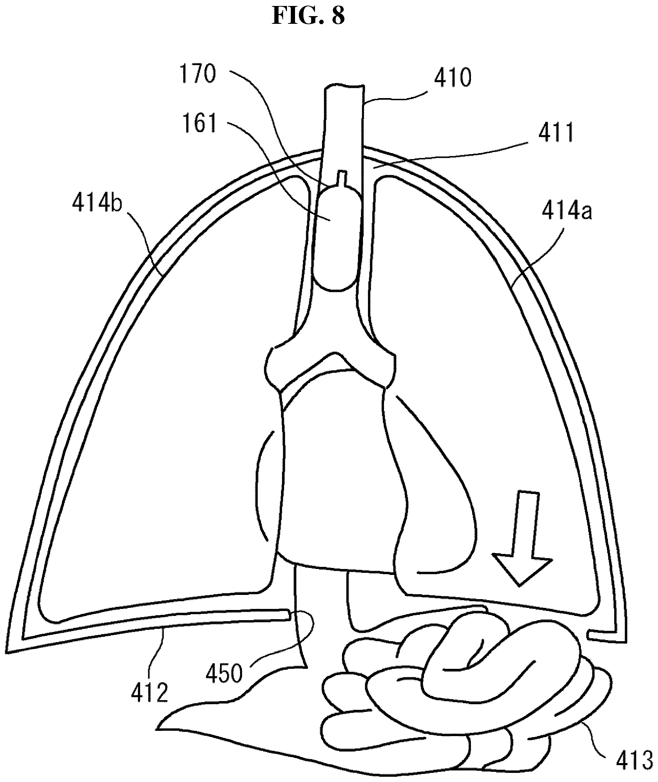

Thereafter, the fetal trachea 410 is kept occluded for a certain period of time. During the time when the trachea 410 is occluded, the pressure of the lung liquid stimulates the growth of the fetal lung 414a and pushes the intraperitoneal organs 413 that entered the thoracic cavity 411 back to the original positions in the peritoneal cavity (see FIG. 8).

The period during which the trachea is occluded is generally about 34 weeks. Approximately after this period, the occlusion of the trachea needs to be terminated in preparation for pulmonary respiration after birth.

### <Termination of the occlusion of the tubular organ>

The user operates the input device 271 of the second ultrasonic wave control unit 222 to cause the ultrasonic device 200 to start displaying a sonogram. This allows the user to have a sonogram including an image of the balloon 170 occluding the fetal trachea 410 displayed on the display 276 by applying the second probe 212 on the proper part of the maternal abdomen 310.

In particular, the transmitter 273 and the receiver 274 receive an instruction from the controller 272 to start displaying the sonogram, based on the operation of the input device 271.

In response to this instruction, the transmitter 273 causes the second probe 212 to emit a second ultrasonic beam 251b by controlling the plurality of second ultrasonic transducers 250b. The plurality of second ultrasonic transducers 250b receive the second ultrasonic beam 251b reflected, for example, inside the maternal body and outputs reception signals in accordance with the received second ultrasonic beam 251b.

The receiver 274 acquires the reception signals from the plurality of second ultrasonic transducers 250b and generates echo data based on the acquired reception signals. The sonogram generator 275 acquires the echo data from the receiver 274 and generates display data based on the acquired echo data. The display 276 acquires the display data from the sonogram generator 275 and displays a sonogram represented by the acquired display data.

Thus, a sonogram of the inside of the maternal body is displayed on the display 276 by applying the second probe 212 to the maternal abdomen 310. The user adjusts the position of the second probe 212 viewing the sonogram to apply the second probe 212 to the proper part of the maternal abdomen 310 and thereby a sonogram including an image of the balloon 170 occluding the fetal trachea 410 is displayed on the display 276.

Note that, for the processing of displaying such a sonogram as described above, various conventional processing techniques may be applied as appropriate.

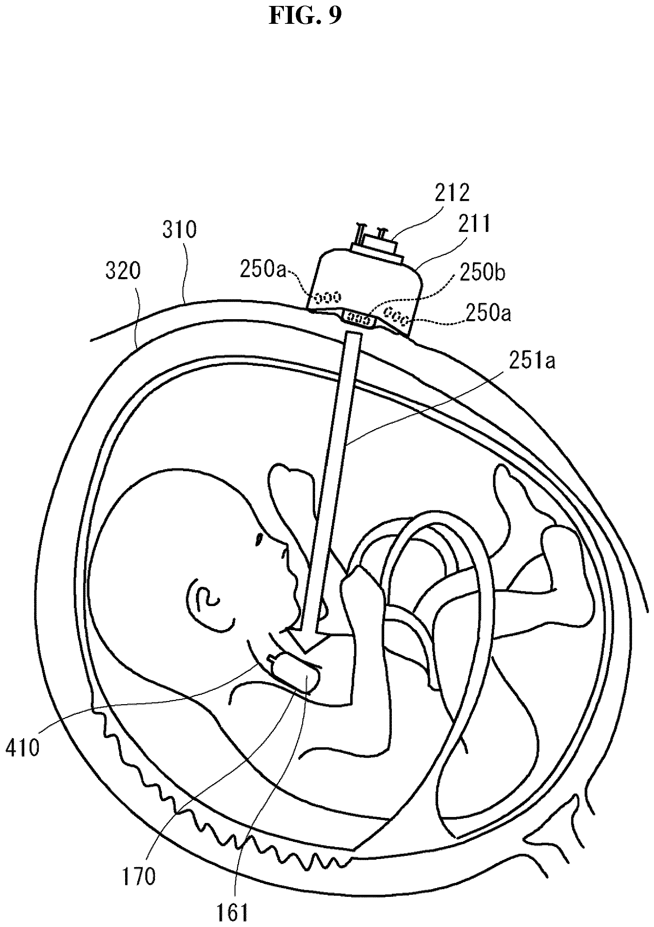

When the balloon 170 is displayed approximately in the center of the sonogram, the user operates the input device 261 of the first ultrasonic wave control unit 221 to cause the first probe 211 to start emitting the first ultrasonic beam 251a as illustrated in FIG. 9. Note that the second ultrasonic beam 251b is not illustrated in FIG. 9 to simplify the drawing.

In particular, the transmitter 263 receives from the controller 262 an instruction to start emitting the first ultrasonic beam 251a, based on the operation of the input device 261. In response to the instruction, the transmitter 263 causes the first probe 211 to emit the first ultrasonic beam 251a by controlling the plurality of first ultrasonic transducers 250a.

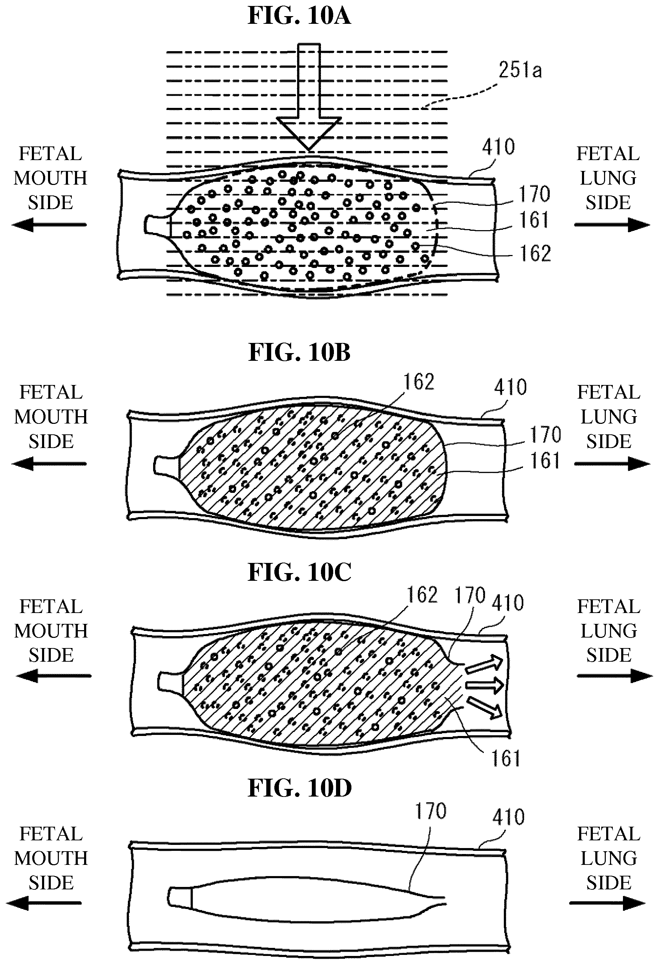

As described above, the plurality of first ultrasonic transducers 250a are arranged inside the sloped surface of the front part of the first probe 211 surrounding the outer circumference of the second probe 212. Therefore, the first ultrasonic beam 251a can be applied to the balloon 170 by causing the first probe 211 to emit the first ultrasonic beam 251a when the balloon 170 is displayed in the center of the sonogram, as illustrated in FIG. 10A, which is an enlarged view of the vicinity of the balloon 170 and the fetal trachea 410.

At this time, the first ultrasonic beam 251a may be applied for example, for a period of 2.5 ms repeatedly every 50 ms at an applied voltage value 200 Vpp, owing to the operation of the plurality of first ultrasonic transducers 250a under the control by the transmitter 263. Such a mode of application of the first ultrasonic beam 251a may be set in advance or may be set by the user operating the input device 261.

When the first ultrasonic beam 251a is applied to the balloon 170, the microcapsules 162 contained in the filling liquid 161 in the balloon 170 are gradually broken and limonene is let out to the filling liquid 161, as illustrated in FIG. 10B. In FIG. 10B, the broken microcapsules 162 are depicted by broken lines and the state in which limonene is let out to the filling liquid 161 is depicted by oblique lines.

Limonene let out to the filling liquid 161 comes into contact with the balloon 170 and the balloon 170 dissolves where the contact is made. As a result, the balloon 170 is broken as illustrated in FIG. 10C. FIG. 10C illustrates an example in which the balloon 170 is broken at the front end thereof (the end closer to the fetal lungs 414a and 414b).

As the filling liquid 161 is let out of the balloon 170 through the broken part, the balloon 170 deflates. This terminates the occlusion of the trachea 410 by the balloon 170 (see FIG. 10D). The deflated balloon 170 is discharged through the fetal mouth to the uterus 320, together with the lung liquid built up in the fetal lungs 414a and 414b due to the occlusion till then.

The user checks that the balloon 170 is broken on the sonogram and causes the first probe 211 to stop emitting the first ultrasonic beam 251a by operating the input device 261 of the first ultrasonic wave control unit 221. The user also causes the second probe 212 to stop emitting the second ultrasonic beam 251b by operating the input device 271 of the second ultrasonic wave control unit 222 and stops the display of the sonogram.

In particular, the transmitter 263 of the first ultrasonic wave control unit 221 receives from the controller 262 an instruction to stop emitting the first ultrasonic beam 251a, based on the operation of the input device 261. In response to the instruction, the transmitter 263 causes the first probe 211 to stop emitting the first ultrasonic beam 251a by controlling the plurality of first ultrasonic transducers 250a.

Further, the transmitter 273 and the receiver 274 of the second ultrasonic wave control unit 222 receive from the controller 272 an instruction to stop displaying the sonogram, based on the operation of the input device 271. In response to the instruction, the transmitter 273 causes the second probe 212 to stop emitting the second ultrasonic beam 251b by controlling the plurality of second ultrasonic transducers 250b and the receiver 274 stops processing such as the receiving the reception signals.

Note that the duration of time in which the first ultrasonic beam 251a is being emitted from the first probe 211 to the balloon 170 before the balloon 170 is broken is, for example, about 1 to several seconds.

As described above, according to the present embodiment, the transmitter 263 of the first ultrasonic wave control unit 221 causes the first probe 211 to emit the first ultrasonic beam 251a that breaks the microcapsules 162 contained in the filling liquid 161 when applied to the balloon 170 filled with filling liquid 161 and occluding the fetal trachea 410. Further, a substance that dissolves the balloon 170 is encapsulated in the microcapsules 162.

This makes it possible to break the microcapsules 162 contained in the filling liquid 161 by applying ultrasonic waves to the balloon 170 filled with the filling liquid 161 and occluding the fetal trachea 410 and to cause the balloon 170 to be dissolved by limonene let out of the broken microcapsules 162.

As described above, the termination of the occlusion of the fetal trachea 410 by the balloon 170 requires no surgical operation and therefore is minimally invasive. Further, unlike endoscopic surgery, it is not necessary to set up a system of medical specialists and the like and therefore it is relatively easy to perform the termination. Further, the balloon 170 can assuredly be broken by limonene. Therefore, it is possible to terminate the occlusion of the fetal trachea 410 by the balloon 170 in a minimally invasive, easy, and assured way.

According to the present embodiment, the user operates the second syringe 160 to inject the filling liquid 161, which is guided through the second catheter 165 to the balloon 170. This allows the balloon 170 to be filled with the filling liquid 161 and to inflate, thereby occluding the fetal trachea 410.

According to the present embodiment, the balloon 170 is made of latex. Further, limonene, which has a property of dissolving rubber such as latex, is used as a substance to dissolve the balloon 170. Limonene is a naturally derived substance and thus very unlikely to adversely affect a human body.

An embodiment of the present disclosure has been described above but the present disclosure is not limited to this embodiment and may be modified, for example, in the following ways.

For example, the embodiment has been described with an example in which the medical system 1 includes an endoscopic device 100 and an ultrasonic device 200 physically separate from each other as illustrated in FIG. 1. However, the medical system may as a whole include the features included by the medical system 1. For example, the first ultrasonic wave control unit 221 and the second ultrasonic wave control unit 222 as well as the endoscope control unit 130 may be integrated physically into one control device that controls the entire medical system. The same advantageous effects as according to the embodiment can be achieved also according to this modified example.

For example, limonene is an example of a substance that dissolves the balloon 170 made of latex. Further, the material for the balloon 170 may be selected as appropriate and a substance that dissolves the balloon 170 made of the material selected as appropriate is preferably encapsulated in the microcapsules 162. A naturally derived essential oil having a property of dissolving rubber such as latex may be used as appropriate as the substance to dissolve the balloon 170. Among the naturally derived essential oils of this kind apart from limonene are, for example, menthol, geraniol, carotenoid (plant pigment), fat-soluble vitamins (vitamins A, D, E, and K), and naturally occurring compounds called terpenoids, which have isoprene as constituent units, such as coenzyme Q. The same advantageous effects as according to the embodiment can be achieved also according to this modified example.

For example, the probe that emits the ultrasonic beam has been described with an example including the first probe 211 and the second probe 212 that separately perform the emissions of the first ultrasonic beam 251a and the second ultrasonic beam 251b, respectively, and other functions. However, a probe that can integrally process the emissions of the first ultrasonic beam 251a and the second ultrasonic beam 251b and other functions may be used. In such a case, the first ultrasonic wave control unit 221 and the second ultrasonic wave control unit 222 may be integrated into one ultrasonic wave control unit. Further, there may be a single first ultrasonic transducer 250a and a single second ultrasonic transducer 250b. Further, all of one or more first ultrasonic transducers 250a and one or more second ultrasonic transducers 250b or at least a part of a plurality of first ultrasonic transducers 250a and a plurality of second ultrasonic transducers 250b may have a plurality of functions at the same time among the function of generating an ultrasonic wave for emitting the first ultrasonic beam 251a, the function of generating an ultrasonic wave for emitting the second ultrasonic beam 251b, and the function of receiving the second ultrasonic beam 251b. The same advantageous effects as according to the embodiment can be achieved also according to this modified example.

For example, according to the embodiment, an example in which the first probe 211 is caused to emit a first ultrasonic beam 251a while the balloon 170 is displayed in the center of the sonogram but the first ultrasonic beam 251a may be emitted to the balloon 170 by designating the position of the balloon 170. In particular, the user may identify the position of the balloon 170 on the basis of the image of the balloon 170 displayed on the display 276 of the second ultrasonic wave control unit 222 and, by operating the input device 261 of the first ultrasonic wave control unit 221, designate the position of the balloon 170 on the transmitter 263 by way of the controller 262. The transmitter 263 may then control the plurality of first ultrasonic transducers 250a in such a way that the first ultrasonic beam 251a is emitted to the designated position. The same advantageous effects as according to the embodiment can be achieved also according to this modified example. Further, as the first ultrasonic beam 251a can be applied assuredly to the balloon 170, the occlusion of the tubular organ by the balloon 170 can be terminated more assuredly.

The foregoing describes some example embodiments for explanatory purposes. Although the foregoing discussion has presented specific embodiments, persons skilled in the art will recognize that changes may be made in form and detail without departing from the broader scope of the invention. Accordingly, the specification and drawings are to be regarded in an illustrative rather than a restrictive sense.

This application claims the benefit of Japanese Patent Application No. 2016-126601, filed on June 27, 2016.

### Reference Signs List

- 1: Medical system
- 100: Endoscopic device
- 110: Sheath
- 111: Sheath body
- 111a: Rear part
- 111b: Front part
- 112: Connecter
- 113, 114: Insertion port part
- 120: Endoscope
- 121: Insertion part
- 122: Operation part
- 123: Wire
- 125: Imaging unit
- 130: Endoscope control unit
- 131: Input device
- 132: Controller
- 133: Imaging controller
- 134: Display image generator
- 135: Display
- 150: First syringe
- 151: Flush liquid
- 155: First catheter
- 156: Spout opening
- 160: Second syringe
- 161: Filling liquid
- 162: Microcapsule
- 165: Second catheter
- 170: Balloon
- 200: Ultrasonic device
- 210: Probe
- 211: First probe
- 212: Second probe
- 220: Control unit
- 221: First ultrasonic wave control unit
- 222: Second ultrasonic wave control unit
- 231, 232: Wire
- 250a: First ultrasonic transducer
- 250b: Second ultrasonic transducer
- 251a: First ultrasonic beam
- 251b: Second ultrasonic beam
- 261: Input device
- 262: Controller
- 263: Transmitter
- 271: Input device
- 272: Controller
- 273: Transmitter
- 274: Receiver
- 275: Sonogram generator
- 276: Display
- 310: Abdomen
- 320: Uterus
- 410: Trachea
- 411: Thoracic cavity
- 412: Diaphragm
- 413: Intraperitoneal organ
- 414a, 414b: Lung
- 450: Hole
- 261: Input device
- 262: Controller
- 263: Transmitter
- 271: Input device
- 272: Controller
- 273: Transmitter
- 274: Receiver
- 275: Sonogram generator
- 276: Display
- 310: Abdomen
- 320: Uterus
- 410: Trachea
- 411: Thoracic cavity
- 412: Diaphragm
- 413: Intraperitoneal organ
- 414a, 414b: Lung
- 450: Hole

## Claims

1. System comprising:
- a balloon (170) comprising latex, and
- a filling liquid (161) for filling the balloon suitable for a method for occluding a tubular organ, the filling liquid comprising a microcapsule (162) in which a naturally derived essential oil, that dissolves the balloon, is encapsulated, wherein the microcapsule is destroyable by an ultrasonic beam; and a liquid that is harmless in the tubular organ and does not dissolve the balloon.

2. The system according to claim 1, wherein the naturally derived essential oil is limonene.

3. The system according to any one of claims 1 or 2, wherein the microcapsule is in a form of a liposome, a macromolecular micelle, or an emulsion.

4. The system according to any one of claims 1 to 3, wherein the filling liquid comprises physiological saline solution.

5. The system according to any one of claims 1 to 4, further comprising a balloon delivery device (100) comprising:
a catheter (165) to which the balloon is attachable at a front end of the catheter; and
injection means (160) for injecting the filling liquid to a tubular tissue through the catheter.

6. The system according to claim 5, wherein the balloon delivery device (100) further comprises an endoscope (120).

7. The system according to any one of claims 1 to 6, further comprising a medical ultrasonic device (200) comprising:
a probe (211) comprising an ultrasonic transducer (250a); and
an ultrasonic wave control unit (221) that controls the ultrasonic transducer to cause the probe to emit an ultrasonic beam to the balloon that is filled with the filling liquid and occludes a tubular organ.

## Patentansprüche

1. System umfassend:
- einem Ballon (170), der Latex aufweist, und
- eine Füllflüssigkeit (161) zum Füllen des Ballons, die für ein Verfahren zum Verschließen eines röhrenförmigen Organs geeignet ist, wobei die Füllflüssigkeit eine Mikrokapsel (162), in der ein natürlich gewonnenes ätherisches Öl, das den Ballon auflöst, eingekapselt ist, wobei die Mikrokapsel durch einen Ultraschallstrahl zerstörbar ist; und eine Flüssigkeit, die in dem röhrenförmigen Organ unschädlich ist und den Ballon nicht auflöst, aufweist.

2. System nach Anspruch 1, wobei das natürlich gewonnene ätherische Öl Limonen ist.

3. System nach einem der Ansprüche 1 oder 2, wobei die Mikrokapsel in Form eines Liposoms, einer makromolekularen Mizelle oder einer Emulsion vorliegt.

4. System nach einem der Ansprüche 1 bis 3, wobei die Füllflüssigkeit physiologische Kochsalzlösung aufweist.

5. System nach einem der Ansprüche 1 bis 4, das ferner eine Ballon abgäbevorrichtung (100) aufweist, mit:
einem Katheter (165), an dem der Ballon an einem vorderen Ende des Katheters befestigbar ist; und Injektionsmittel (160) zum Injizieren der Füllflüssigkeit in ein röhrenförmiges Gewebe durch den Katheter.

6. System nach Anspruch 5, wobei die Ballonabgabevorrichtung (100) ferner ein Endoskop (120) aufweist.

7. System nach einem der Ansprüche 1 bis 6, ferner umfassend eine medizinische Ultraschallvorrichtung (200), umfassend:
eine Sonde (211) mit einem Ultraschallwandler (250a); und
eine Ultraschallwellen-Steuereinheit (221), die den Ultraschallwandler steuert, um zu bewirken, dass die Sonde einen Ultraschallstrahl an den Ballon aussendet, der mit der Füllflüssigkeit gefüllt ist und ein rohrförmiges Organ verschließt.

## Revendications

1. Un système comprenant :
- un ballon (170) comprenant du latex, et
- un liquide de remplissage (161) destiné à remplir le ballon et adapté
pour un procédé d'occlusion d'un organe tubulaire, le liquide de remplissage comprenant une microcapsule (162) dans laquelle est encapsulée une huile essentielle d'origine naturelle qui dissout le ballon, dans lequel la microcapsule peut être détruite par un faisceau ultrasonore ; et un liquide qui est inoffensif dans l'organe tubulaire et ne dissout pas le ballon.

2. Le système selon la revendication 1, dans lequel l'huile essentielle d'origine naturelle est le limonène.

3. Le système selon l'une quelconque des revendications 1 ou 2, dans lequel la microcapsule se présente sous la forme d'un liposome, d'une micelle macromoléculaire ou d'une émulsion.

4. Le système selon l'une quelconque des revendications 1 à 3, dans lequel le liquide de remplissage comprend une solution saline physiologique.

5. Le système selon l'une quelconque des revendications 1 à 4, comprenant en outre un dispositif d'administration à ballon (100) comprenant :
un cathéter (165) auquel le ballon peut être fixé à une extrémité avant du cathéter ; et
des moyens d'injection (160) pour injecter le liquide de remplissage dans un tissu tubulaire à travers le cathéter.

6. Le système selon la revendication 5, dans lequel le dispositif d'administration à ballon (100) comprend en outre un endoscope (120).

7. Le système selon l'une quelconque des revendications 1 à 6, comprenant en outre un dispositif médical ultrasonore (200) comprenant :
une sonde (211) comprenant un transducteur ultrasonore (250a) ; et
une unité de commande d'ondes ultrasonores (221) qui commande le transducteur ultrasonore pour amener la sonde à émettre un faisceau ultrasonore vers le ballon qui est rempli du liquide de remplissage et occlut un organe tubulaire.
